Europäisches Patentamt

European Patent Office

Office européen des brevets

(11) Veröffentlichungsnummer: **0 303 277**
**A1**

## (12) EUROPÄISCHE PATENTANMELDUNG

(21) Anmeldenummer: 88113092.6

(51) Int. Cl.⁴: **A61K 35/78**

(22) Anmeldetag: 11.08.88

(30) Priorität: 12.08.87 DE 3726864

(43) Veröffentlichungstag der Anmeldung:
15.02.89 Patentblatt 89/07

(84) Benannte Vertragsstaaten:
AT BE CH DE ES FR GB GR IT LI LU NL SE

(71) Anmelder: **OXO CHEMIE AG**
**Ch. des Primevères 45**
**CH-1701 Fribourg(CH)**

(72) Erfinder: **Bauer, Johan, Dr. Dr.med.**
**Volkartstrasse 64**
**D-8000 München 19(DE)**

(74) Vertreter: **Spott, Gottfried, Dr. et al**
**Patentanwälte Spott und Puschmann**
**Sendlinger-Tor-Platz 11**
**D-8000 München 2(DE)**

(54) Verwendung von Ginkgo bilobae Extrakten zur Bekämpfung von entzündlichen Prozessen.

(57) Die Erfindung betrifft die Verwendung von Extrakten aus getrockeneten Ginkgo biloba Blättern bzw. deren Inhaltsstoffen, nämlich den Ginkgoliden, zur Bekämpfung von entzündlichen Prozessen.

EP 0 303 277 A1

## Verwendung von Ginkgo bilobae Extrakten zur Bekämpfung von entzündlichen Prozessen

Die Erfindung betrifft eine neue Verwendung von Extrakten aus getrockneten Ginkgo bilobae Blättern bzw. deren Inhaltsstoffen, nämlich den Ginkgoliden, gegen entzündliche Prozesse.

Bei der Behandlung von entzündlichen Prozessen, wie sie insbesondere als Folgeerscheinungen von Verbrennungen auftreten und mit Störungen der Blutbestandteile, insbesondere der Thrombozyten, einhergehen, wurde, beispielsweise gemäß EP-A-0 169 466 bereits vorgeschlagen, eine Kombination von Xanthinderivaten mit Acetylsalicylsäurederivaten einzusetzen. Diese Kombition zeigte sich, insbesondere wenn sie in zeitlicher Abfolge gegeben wurde, jedoch als wirksam gegen Störungen des Gleichgewichts der Blutbestandteile, wie sie beispielsweise bei Verbrennungen auftreten.

Diese bekannte Kombination ist jedoch in Fällen, bei denen eine Acetylsalicylsäureunverträglichkeit oder auch eine Unverträglichkeit der Xanthinderivate auftritt, nicht oder schlecht einzetzbar.

Es ist daher Aufgabe der Erfindung, ein Präparat zu schaffen, das die Entzündungskaskade bzw. die biochemischen entzündlichen Reaktionen, wie sie beispielsweise bei einer akuten Verbrennung entstehen, hemmt und auch in Fällen eingesetzt werden kann, in denen eine Unverträglichkeit der aus der EP-A-0 169 466 bekannten Kombinationspräparate auftritt, bei dem zudem eine Beachtung irgendwelcher zeitlicher Abfolge unnötig ist. Es dient auch der perioperativen Entzündungsprophylaxe und damit als perioperative Infektionsprophylaxe. Es läßt Schmerzmittel und Antibiotika einsparen, die als perioperative Prophylaxe oder bei eintretendem Infekt gegeben werden. Auch als Mittel gegen den Sonnenbrand ist es parenteral, oral oder topisch einsetzbar.

Die Aufgabe wird erfindungsgemäße durch die Verwendung von Extrakten aus getrockeneten Ginkgo bilobae Blättern bzw. deren Inhaltsstoffen, nämlich den Ginkgoliden, gegen entzündliche Prozesse gelöst.

Dabei kann der Wirkstoff, nämlich der Ginkgoextrakt bzw. die Ginkgolide in einer Injektionslösung gegebenenfalls mit einem üblichen Trägermaterial vorliegen.

Es kann auch bevorzugt sein, daß der Ginkgoextrakt bzw. die Ginkgolide in einer oral verabreichbaren Form vorliegen, wie einer Trinkampulle, Dragees oder Kapseln.

Erfindungsgemäß sind typische entzündliche Prozesse, die durch den erfindungsgemäßen Wirkstoff günstig beeinflußbar sind, die Erstbehandlung von Verbrennungen (eingeschlossen Sonnenbrand), Verbrühungen und Strahlenschäden sowie sogenannte Kombinationstraumata, Erfrierungen, Schockzustände, Sepsis, Pankreatitis, Tourniquet Syndrom und Palacosreaktion.

Dabei wird unter Ginkgolid hier der wirksame Bestandteil des Extr. Ginkgo bilobae, der die Thrombozytenaggregation beeinflußt und unter Ginkgoextrakt wird ein Extrakt aus getrockneten Ginkgo bilobae Blättern verstanden, wie er beispielsweise von der Firma Dr. Willmar Schwabe, Karlsruhe, Bundesrepublik Deutschland, unter dem Warenzeichen TEBONIN® im Handel erhältlich ist.

Das erfindungsgemäße Kombinationspräparat liegt zur schnellen Verabreichung bevorzugt in einer Injektionslösung, ggf. mit einem Trägermaterial, vor, kann aber für spezielle Lagerbedingungen (wie in Erste-Hilfe-Kästen) auch in Tablettenform bei geeigneten Krankheitsbildern bzw. Verfügbarkeit gegeben werden.

Insbesondere eignet sich das erfindungsgemäße Präparat zur Verwendung bei der Erstbehandlung von Verbrennungen für medizinische und tiermedizinische Zwecke und bei allen Schäden, die ohne Rücksicht auf das verursachende Läsionsprinzip, die Entzündungskaskade in Gang setzen.

Es hat sich nun überraschenderweise gezeigt, daß der Wirkstoff, der bisher als die zerebrale und periphere Mangeldurchblutung und Mangelernährung verhinderndes Präparat im Handel war, auch für verschiedene Teilbereiche der bei entzündlichen Prozessen ablaufenden Entzündungskette mit einem die Thrombozytenaggregation (Platelet activating factor wird gehemmt) hemmenden Wirkstoff, wie Gingkoextrakt (beispielsweise unter dem Warenzeichen RÖKAN® der Firma Intersan, Ettlingen, Bundesrepublik Deutschland, oder TEBONIN® der Firma W. Schwabe, Karlsruhe, Bundesrepublik Deutschland im Handel als durchblutungsförderndes Agens) bei Untersuchungen an Schweinen mit Verbrennungen, aber auch bei menschlichen Opfern von Verbrennungen/Schockzuständen bei akuter Gabe dieser Wirkstoffe, bevorzugt in Form einer "One Shot-Injection" das Auftreten von teilweise letalen Spätfolgen überschießender biochemischer entzündungsartiger Reaktionen, wie ARDS (Lungenschäden), Verbrennungskrankheit, aber auch Sepsis verhindern und auch bei Pankreatitis, akutem Herzinfarkt und allen anderen akuten Ereignissen, die auf einem völligen Ungleichgewicht des Mediatorenspiegels beruhen, abzufangen und therapeutisch zu steuern vermögen.

Es wird angenommen, daß es sich hier um ein frühes Eingreifen in die Entzündungskette unter anderem durch die Hemmung von PAF (Platelet Activating Factor) durch den Ginkgo biloba Extrakt bzw. dessen wirksame Bestandteile sowie um Cyclooxygenasehemmung oder Lipoxygenasehemmung (Inhibierung eines Teils der Arachidonsäurekaskade) je nach eingesetztem Entzündungshemmer, handelt.

Nachfolgend soll die Erfindung anhand eines Verbrennungsversuchsbeispiels, das als typisch für eine physikalisch - ohne bakterielle oder virale Komponenten -verursachte Entzündung betrachtet wird, näher erläutert werden, das die überraschende Wirkung des erfindungsgemäßen Präparates zeigt.

Versuch:

1 deutsches Hausschwein, 20 kg, weiblich, wurde mit VETRANQUIL® und NACOSEN® i. v. über die Ohrvene betäubt und an der Flanke 10 Sekunden in 75 Grad Celsius heißem Wasser verbrüht, was zu einer standardisierten tiefdermalen Verbrennung des Grades 2b führt.

5 Minuten post läsionem erhielt das Tier 25 ml entsprechend 87,5 mg eines auf 4,2 mg Ginkgoflavonglykoside standardisierten Ginkgoblätterextraktes, wie er unter dem Warenzeichen TEBONIN® im Handel erhältlich ist, über einen iugularis interna Katheter.

1 Stunde nach Applikation des Mittels sieht man - im Gegensatz zum Kontrolltier - daß sich die Läsion blau-livide verfärbt. 3 Stunden später erhielt das Tier auf die gleiche Weise eine zweite Dosis (i.v.) sowie am ersten bis 4. Tag p.l. täglich eine Dosis i.v.. Am 7. Tag post läsionem ist die Läsion makroskopisch und im histologischen Präparat auf dem Wege der Heilung, während beim Kontrolltier noch keine Heilung zu verzeichnen ist.

Im täglich abgenommenen Indomethazin-Plasma des behandelten Tieres konnten keine Prostanoide gemessen werden, die über dem Hintergrundrauschen liegen, während bei der Kontrollgruppe insbesondere 4 Stunden post läsionem und am 3. Tag post läsionem sehr hohe Prostanoidwerte gemessen werden. Daruas ist ersichtlich, daß der Ginkgo-Extrakt unerwartet positive Wirkungen bei durch Verbrennungen hervorgerufenen entzündlichen Prozessen zeigt.

Durch das neue Kombinationspräparat wird die Startstrecke jedweder Entzündungsreaktionen - unabhängig vom Auslösemechanismus - so gestört, daß die kritische Masse an Mediatoren nicht erreicht wird und die letale, unkontrollierte und unkontrollierbare Kettenreaktion zur gesteuerten, behebbaren Störung des Mediatorenspiegels gezügelt bzw. gebremst wird, mit der ein Organismus fertig werden kann.

## Ansprüche

1. Verwendung von Extrakten aus getrockneten Ginkgo biloba Blättern bzw. deren Inhaltsstoffen, den Ginkgoliden, zur Bekämpfung von entzündlichen Prozessen.

2. Verwendung nach Anspruch 1, dadurch gekennzeichnet, daß der Ginkgoextrakt bzw. die Ginkgolide in einer Injektionslösung gegebenenfalls mit einem Trägermaterial, vorliegt.

3. Verwendung nach Anspruch 1, dadurch gekennzeichnet, daß der Ginkgoextrakt bzw. die Ginkgolide in einer oral verabreichbaren Form vorliegen, wie einer Trinkampulle, Dragees oder Kapseln.

4. Verwendung nach einem der vorangehenden Ansprüche für die Erstbehandlung von Verbrennungen (eingeschlossen Sonnenbrand), Verbrühungen und Strahlenschäden sowie sogenannte Kombinationstraumata, Erfrierungen, Schockzustände, Sepsis, Pankreatitis, Tourniquet Syndrom und Palacosreaktion.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | CHEMICAL ABSTRACTS, Band 107, 1987, Seite 324, Zusammenfassung Nr. 28395j, Columbus, Ohio, US; & JP-A-62 33 118 (KURARAY CO., LTD) 13-02-1987 * Zusammenfassung * --- | 1-4 | A 61 K 35/78 |
| X | GB-A-2 162 062 (SOCIETE DE CONSEILS DE RECHERCHES ET D'APPLICATIONS SCIENTIFIQUES) * Ansprüche 1,2,3,4,8,10; Seite 1, Zeilen 11-17 * --- | 1-4 | |
| X | THE LANCET, Band 1, 31. Januar 1987, Seiten 248-251; K.F. CHUNG et al.: "Effect of a ginkgolide mixture (BN 52063) in antagonising skin and platelet responses to platelet activating factor in man" * Seite 248, Spalte 1,; Seiten 249,250 * ----- | 1-4 | |

**RECHERCHIERTE SACHGEBIETE (Int. Cl.4)**

A 61 K

Der vorliegende Recherchenbericht wurde für alle Patentansprüche erstellt

| Recherchenort | Abschlußdatum der Recherche | Prüfer |
|---|---|---|
| DEN HAAG | 24-11-1988 | PEETERS J.C. |

EPO FORM 1503 03.82 (P0403)